# FASCICULE DE BREVET EUROPEEN

(11) **EP 0 431 998 B1**
(45) Date de publication et mention de la délivrance du brevet: **30.05.2001**
(21) Numéro de dépôt: 90403246.3
(22) Date de dépôt: 16.11.1990
(51) Int. Cl.: C01B 21/16, B01D 3/06

(54) **Procédé de vaporisation d'une solution d'hydrate d'hydrazine**
Verfahren zur Verdampfung einer Hydrazinhydratlösung
Process for evaporation of a hydrazine hydrate solution

(30) Priorité: 04.12.1989 FR 8915968
(43) Date de publication de la demande: 12.06.1991
(60) Demande divisionnaire: 99111127.9
(73) Titulaire: Atofina, 92800 Puteaux (FR)
(72) Inventeur: Krempf, Gérard, F-69003 Lyon (FR); Collier, Bertrand, F-65250 La Barthe de Neste (FR); Tellier, Pierre, F-69110 Sainte-Foy-Les-Lyon (FR); Pleuvry, Jean-Pierre, F-65250 La Barthe de Neste (FR)

(56) Documents cités:
- DE-A- 2 536 918
- DE-B- 1 058 030
- FR-A- 1 548 921
- FR-A- 2 323 635
- R. Billet: "Evaporation Technology" mai 1989, VCH Verlagsgesellschaft, Weinheim (BRD)
- OIL AND GAS JOURNAL. vol. 75, no. 37, septembre 1977, TULSA US pages 101 - 102; J.R. Pudlock: "Corrosion-resistant shell and tube exchangers costs compared"
- HEATING, PIPING AND AIR CONDITIONING. vol. 59, no. 12, décembre 1987, STAMFORD US pages 81 - 82; Z.H. Ayub: "Tubeside erosion/corrosion in heat exchangers."

## Description

La présente invention concerne un procédé de vaporisation d'une solution d'hydrate d'hydrazine.

La production industrielle de l'hydrate d'hydrazine se fait selon les procédés RASCHIG, BAYER et ATOCHEM.

Dans le procédé RASCHIG on oxyde l'ammoniac par un hypochlorite pour obtenir une solution diluée d'hydrate d'hydrazine qu'il faut ensuite concentrer par distillation.

Le procédé BAYER est une variante du procédé RASCHIG qui consiste à déplacer un équilibre chimique en piégeant, à l'aide d'acétone, l'hydrazine formée sous forme d'azine (CH₃)₂C=N-N=C(CH₃)₂. L'azine est ensuite isolée puis hydrolysée en hydrate d'hydrazine.

Le procédé ATOCHEM consiste à oxyder un mélange d'ammoniac et d'une cétone par l'eau oxygénée en présence d'un catalyseur pour faire directement l'azine qu'il suffit ensuite d'hydrolyser en hydrate d'hydrazine. Le procédé ATOCHEM est décrit dans de nombreux brevets, par exemple US-A-3 972 878, US-A-3 972 876, US-A-3 948 902 et US-A-4 093 656.

L'hydrolyse d'une azine en hydrate d'hydrazine est décrite dans les brevets US-A-4 724 133 SCHIRMANN et al, US-A-4 725 421 SCHIRMANN et al et GB-A-1 164 460. Cette hydrolyse s'effectue dans une colonne de distillation qu'on alimente avec de l'eau et de l'azine, en tête on récupère la cétone et en pied l'hydrate d'hydrazine. Dans le fond d'une colonne d'hydrolyse comme dans le fond d'une colonne de concentration d'hydrate d'hydrazine, il existe un dispositif pour vaporiser l'hydrate d'hydrazine.

La demanderesse a constaté que si on utilise un rebouilleur à thermosiphon, un serpentin noyé dans le fond de la colonne ou un faisceau tubulaire noyé dans le fond de la colonne et en règle générale un rebouilleur où la vaporisation se produit au contact de la surface de chauffe, on observe une décomposition de l'hydrate d'hydrazine. La demanderesse a découvert que si on vaporise l'hydrate d'hydrazine par détente et non pas au contact de la surface de transfert de l'énergie thermique, on peut réduire sensiblement la décomposition.

La présente invention est donc un procédé de vaporisation d'une solution d'hydrate d'hydrazine tel que défini dans la revendication 1 consistant à chauffer cette solution essentiellement en phase liquide puis à détendre cette solution.

La solution d'hydrate d'hydrazine peut être une solution d'eau et d'hydrazine en proportions comprises entre l'hydrazine (ou l'hydrate) très diluée dans l'eau et l'hydrate d'hydrazine. La solution d'hydrate d'hydrazine peut être une solution contenant aussi une azine, une hydrazone, des cétones ; cette solution pouvant être aqueuse ou anhydre.

On désigne respectivement par azine et hydrazone les produits de formule : 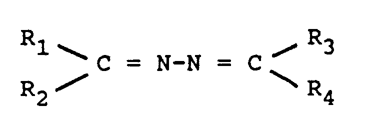 et 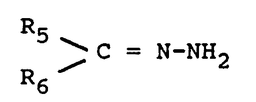 dans lesquels R₁ à R₆ sont identiques ou différents et désignent de l'hydrogène, un radical alkyl linéaire ayant de 1 à 12 atomes de carbone, un radical alkyl ramifié ou cycloalkyl ayant de 3 à 12 atomes de carbone, un radical aryl ayant de 6 à 12 atomes de carbone. Les radicaux R₁ à R₆ reliés au même atome de carbone de l'azine ou de l'hydrazone peuvent être eux-mêmes reliés et représenter ensemble un radical alkylène linéaire ou ramifié ayant de 3 à 12 atomes de carbone.

Tous les radicaux R₁ à R₆ précédents peuvent aussi être substitués par un chlore, un brome, un fluor ou un groupe nitro, hydroxy, alcoxy ou une fonction ester. L'invention est particulièrement utile pour l'acétone azine :

CH₃(CH₃)C=N-N=C(CH₃)CH₃

la méthyléthylcétazine C₂H₅(CH₃)C=N-N=C(CH₃)C₂H₅, et les hydrazones correspondantes.

Pour chauffer cette solution essentiellement en phase liquide, il suffit de mettre la phase liquide en pression pendant qu'on la chauffe, c'est-à-dire que la solution d'hydrate d'hydrazine absorbe l'énergie thermique sous forme d'une augmentation de sa température puis on détend cette solution et l'énergie précédente est restituée sous forme d'une vaporisation.

On ne sortirait pas du cadre de l'invention si une partie de l'énergie thermique était consommée par une réaction d'hydrolyse d'une azine et/ou d'une hydrazone en hydrazine ou hydrazone correspondante.

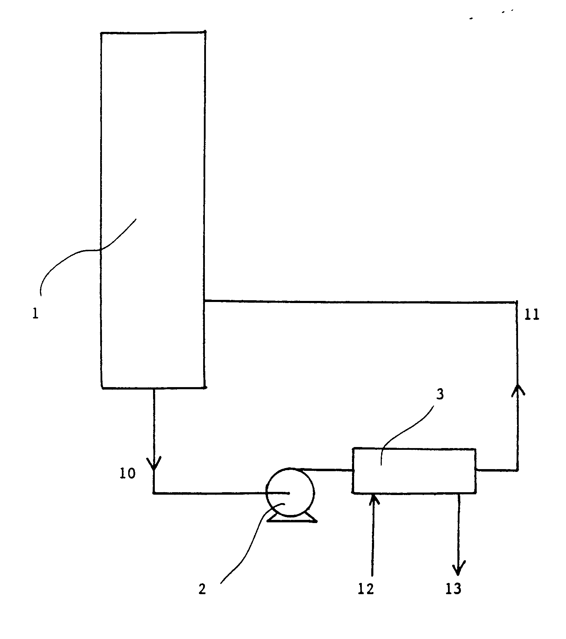

On ne sortirait pas du cadre de l'invention si pendant le chauffage de la solution d'hydrate d'hydrazine, une fraction était vaporisée, cette fraction pouvant représenter jusqu'à 3 ou 5 % en poids de la solution qui est chauffée puis détendue. La figure 1 décrit un mode de réalisation préféré de l'invention.

Dans cette figure 1, 1 représente une colonne à distiller, 2 une pompe et 3 un échangeur de chaleur. La solution d'hydrate d'hydrazine en pied de la colonne 1 est transportée via le tuyau 10 par la pompe 2 à travers l'échangeur 3 puis retourne dans la colonne par la tuyauterie 11. Par simplification on n'a pas représenté le soutirage de pied de la colonne 1 qui peut être par exemple au refoulement de 2. La pompe 2 permet de donner à la solution d'hydrate d'hydrazine une pression supérieure à celle du fond de la colonne 1 lui permettant de circuler dans l'échangeur 3 et le tuyau 11. Cette pression permet de maintenir la solution en phase essentiellement liquide dans l'échangeur 3. L'échangeur 3 est alimenté en 12 par de la vapeur ou un fluide caloporteur qui est évacué en 13. On peut disposer en un point quelconque du tuyau 11 un dispositif pour créer une perte de charge, tel qu'une vanne ou un diaphragme. L'homme de métier peut choisir facilement de mettre ou de ne pas mettre un dispositif de perte de charge sur le tuyau 11 en fonction des pertes de charge créées par l'échangeur 3 et la tuyauterie 11. La pression relative fournie par la pompe, c'est-à-dire la différence de pression entre le refoulement de la pompe et le pied de la colonne peut être quelconque mais elle est habituellement supérieure à 0,5 bar et de préférence comprise entre 1 et 10 bars.

On ne sortirait pas du cadre de l'invention en disposant l'échangeur 3 et la pompe 2 très en-dessous du niveau du fond de la colonne 1. On créé ainsi une pression supplémentaire sur la solution d'hydrate d'hydrazine dans l'échangeur 3. On pourrait aussi selon cette variante si la différence de niveau est suffisante pour maintenir dans l'échangeur 3 la solution en phase liquide, supprimer la pompe 2. Cette forme de réalisation n'a d'intérêt que si la colonne 1 est à une hauteur suffisante au-dessus du sol.

La colonne 1 peut être aussi un simple réservoir dans lequel on effectue une réaction nécessitant une vaporisation et qui serait munie d'une alimentation et éventuellement d'un soutirage en tête avec ou sans reflux. Cette colonne 1 étant insérée éventuellement dans un procédé. C'est par exemple un réservoir dans lequel on effectue une réaction endothermique.

La présente invention vise à vaporiser des solutions aqueuses d'hydrate d'hydrazine se trouvant à des températures comprises entre 130 et 220°C, et une pression supérieure à la pression atmosphérique. L'invention est ainsi particulièrement utile pour le bouilleur en pied d'une colonne d'hydrolyse d'azine ou d'hydrazone en hydrazine.

La présente invention concerne aussi l'utilisation de matériaux particuliers pour réaliser la surface d'un dispositif de vaporisation d'une solution d'hydrate d'hydrazine.

La demanderesse a découvert que la décomposition de l'hydrazine varie selon le matériau qui constitue la surface de transfert de l'énergie de vaporisation et que cette propriété était valable aussi bien (i) pour une vaporisation consistant à chauffer la solution d'hydrate d'hydrazine essentiellement en phase liquide puis à détendre cette solution que (ii) pour une vaporisation conventionnelle dans un bouilleur à thermosiphon, un serpentin noyé et en général tout dispositif dans lequel la vaporisation se produit au contact de la surface de chauffe.

La demanderesse a ainsi découvert qu'on peut classer dans l'ordre le titane, les oxydes de chrome, l'aluminium, l'acier ordinaire, l'inox 304, l'inox 316, le nickel et ses alliages et le zirconium. Le titane provoquant moins de décomposition que le zirconium. L'inox 304 et 316 sont les désignations usuelles des aciers inoxydables selon la norme AISI (American Iron and Steel Institute).

Il est clair que le matériau du dispositif de vaporisation d'une solution d'hydrazine concerne le matériau au contact de la solution d'hydrate d'hydrazine et non pas le matériau au contact du fluide de chauffage. Les oxydes de chrome sont utilisés de préférence sous forme de revêtement d'une surface métallique.

### EXEMPLE

Une capacité de volume 300 l contenant une solution d'hydrate d'hydrazine est munie d'un échangeur de chaleur extérieur de 5 m² disposé verticalement avec retour dans la partie supérieure de la capacité. On peut aussi disposer une pompe pouvant débiter 20 m³/heure avec une hauteur manométrique de 2 bars entre la capacité et l'échangeur et un disphragme en sortie pour le faire fonctionner en bouilleur à circulation forcée. On condense 250 Kg/h de vapeur à 18 bars dans l'échangeur. La capacité est munie d'un condenseur de reflux total pour éliminer l'énergie fournie à l'échangeur et d'un dégazage pour recueillir l'azote, l'hydrogène et l'ammoniac provenant de la décomposition de l'hydrazine et ainsi mesurer cette décomposition.

L'ensemble est construit en acier et on a essayé des échangeurs en différents matériaux.

Pour tester chaque matériau, on remplit la capacité avec 50 kg d'hydrate d'hydrazine (N₂H₄, H₂O) dilué dans 250 kg d'eau, puis on envoie de la vapeur 18 bars pour faire fonctionner l'échangeur en bouilleur (on condense 250 kg/heure). On mesure au bout d'une heure la quantité d'hydrazine restant dans la capacité. Cette valeur est corellée par la mesure du dégazage. La pression dans la capacité est de 9 bars relatifs et la température 180°C.
**a) On fonctionne en thermosiphon**
   Si on utilise un échangeur en inox 316, il reste au bout d'une heure 45 kg d'hydrate d'hydrazine, pour l'acier il en reste 47 kg et pour le titane 48,5 kg.
**b) On fonctionne avec la pompe**
   En utilisant un échangeur en inox 316 il reste au bout d'une heure 47,5 kg d'hydrate d'hydrazine.

## Revendications

1. Procédé de vaporisation d'une solution d'hydrate d'hydrazine se trouvant à une pression supérieure à la pression atmosphérique et une température comprise entre 130 et 220°C consistant à chauffer cette solution essentiellement en phase liquide, puis à détendre cette solution.

2. Procédé selon la revendication 1, caractérisé en ce que la quantité vaporisée pendant le chauffage est inférieure à 5 % de la quantité chauffée.

3. Procédé selon la revendication 1 ou 2, caractérisé en ce que :
- on prélève la solution dans le fond d'une colonne ;
- on la transfère par une pompe dans un échangeur de chaleur ;
- puis on retourne cette solution dans le fond de la colonne.

4. Procédé selon l'une des revendications 1 à 3, caractérisé en ce qu'on l'utilise pour le rebouilleur d'une colonne d'hydrolyse d'azine ou d'hydrazone en hydrazine.

5. Procédé selon l'une des revendications 1 à 4, caractérisé en ce que la surface de chauffe est en titane ou en oxyde de chrome.

## Patentansprüche

1. Verfahren zur Verdampfung einer Hydrazinhydrat-Lösung, die sich bei einem Druck oberhalb des Atmosphärendrucks und einer Temperatur im Bereich von 130 bis 220 °C befindet, das darin besteht, diese Lösung im wesentlichen in flüssiger Phase zu erhitzen und anschließend diese Lösung zu entspannen.

2. Verfahren nach Anspruch 1, dadurch gekennzeichnet, daß die Flüssigkeitsmenge, die beim Erhitzen verdampft, weniger als 5 % der erhitzten Flüssigkeitsmenge entspricht.

3. Verfahren nach Anspruch 1 oder 2, dadurch gekennzeichnet, daß
- die Lösung am Boden einer Kolonne entnommen wird,
- die entnommene Lösung mit einer Pumpe in einen Wärmetauscher geleitet wird und
- anschließend diese Lösung zum Boden der Kolonne zurückgeführt wird.

4. Verfahren nach einem der Ansprüche 1 bis 3, dadurch gekennzeichnet, daß es für den Siedekessel einer Kolonne, worin ein Azin oder ein Hydrazon zu Hydrazin hydrolysiert wird, verwendet wird.

5. Verfahren nach einem der Ansprüche 1 bis 4, dadurch gekennzeichnet, daß die Heizfläche aus Titan oder Chromoxid besteht.

## Claims

1. Process for the evaporation of a hydrazine hydrate solution which is at a pressure greater than atmospheric pressure and a temperature between 130 and 220°C, which consists in heating this solution essentially in the liquid phase and in then reducing the pressure of this solution.

2. Process according to Claim 1, characterized in that the amount evaporated during the heating is less than 5% of the amount heated.

3. Process according to Claim 1 or 2, characterized in that:
- the solution is withdrawn from the bottom of a column;
- it is transferred via a pump into a heat exchanger;
- then this solution is returned to the bottom of the column.

4. Process according to one of Claims 1 to 3, characterized in that it is used for the reboiler of a column for the hydrolysis of azine or of hydrazone to hydrazine.

5. Process according to one of Claims 1 to 4, characterized in that the heating surface is made of titanium or is made of chromium oxide.
